# EUROPEAN PATENT APPLICATION

(11) **EP 4 566 614 A1**
(43) Date of publication of application: **11.06.2025**
(21) Application number: 23850150.6
(22) Date of filing: 03.08.2023
(51) Int. Cl.: A61K 31/7115, A61K 31/42, A61K 31/436, A61K 31/519, A61K 31/57, A61K 38/13, A61K 47/60, A61P 27/02, C12N 15/115

(54) **DRY EYE REMEDY CONTAINING DNA OLIGONUCLEOTIDE SELECTIVELY BINDING TO IFN-y**

(30) Priority: 03.08.2022 JP 2022123810
(71) Applicant: TAGCyx Biotechnologies Inc., Tokyo 1530041 (JP)
(72) Inventor: HORI, Miyuki, Tokyo 153-0041 (JP)
(74) Representative: RGTH
(86) International application number: PCT/JP2023/028454
(87) International publication number: WO 2024/029597

(57) **Abstract**

Provided is a dry eye therapeutic agent that can selectively inhibit IFN-y, have no risk of biological contamination, and can be stored at room temperature. Provided is a dry eye therapeutic agent, comprising as an active ingredient a DNA oligonucleotide having a nucleotide sequence set forth in any of SEQ ID NO:1 to 3 and selectively binding to IFN-y. A base, X, in the sequence of the DNA oligonucleotide having the nucleotide sequence set forth in SEQ ID NO: 3 is an artificially produced base, and the artificially produced base may be chemically modified with a low-molecular-weight compound, a medium-molecular-weight compound, a large-molecular-weight compound, a biopolymer, or a biocompatible polymer.

## Description

### [Technical Field]

The present invention relates to a dry eye therapeutic agent containing an DNA oligonucleotide that selectively binds to IFN-y.

### [Background Art]

Dry eye is a generic term for eye diseases caused by the dryness of the eye surface. Dry eye is a disease in which the stability of the tear layer is deteriorated due to various factors, and is defined as follows: Discomfort or visual dysfunction is caused, and the disorder of the eye surface may be accompanied thereby (NPL 1). If the symptoms are exacerbated, dry eye may require corneal transplantation or lead to blindness. The number of dry eye patients in Japan is estimated to be 22 million or more by the Japanese Ophthalmological Society (NPL 2), and tends to increase. In the mechanism of dry eye development, a "deterioration in the stability of the tear layer" is important. The tear layer is unstabilized due to a decrease in the amount of tears, a rise in the surface tension of tears, an increase in the evaporation of tears, and a deterioration in the hydrophilicity (wettability) of the corneal/conjunctival epithelium. These are clinically considered to be aqueous-deficient dry eye, dry eye accompanied by meibomian gland dysfunction, dry eye due to environmental factors such as prolonged use of video display terminals, and dry eye due to abnormal mucin on the eye surface.

Aqueous-deficient dry eye is further classified into a type accompanied by the structural abnormality of the lacrimal glands (so-called Sjogren's syndrome (SS) type) and a type not accompanied by apparent tissue abnormality (non-SS type). The SS type dry eye is an autoimmune disease in which chronic inflammation occurs in systemic exocrine glands including lacrimal glands and salivary glands. The inflammation leads to lacrimal gland disorder, resulting in the development of serious dry eye. In non-SS type dry eye, a deterioration in the stability of the tear layer causes dryness, resulting in the epithelial disorder of the cornea/conjunctiva. Consequently, the wettability of the epithelial surface is deteriorated to further unstabilize the tear layer. Such a vicious cycle is supposed to cause inflammation to promote epithelial disorder (NPL 3).

Any fundamental therapy for dry eye has not been found yet. Clinical Guidelines for Dry Eye (NPL 1) exemplifies, as methods for improving the symptoms, palliative therapies such as artificial tears for tear supplementation, hyaluronic acid instillation, and the instillation treatment of rebamipide (Mucosta (R)) and diquafosol sodium (Diquas(R)) and surgical treatment such as punctal plug insertion in which in order to prevent the excretion of tears, punctal are blocked with plugs, resulting in the maintenance of the amount of tears. Since these are therapies for improving symptoms of dry eye, not fundamental therapies, these are disadvantageous in that the therapy cessation leads to the recurrence of the symptoms.

In Japan, dry eye is defined as "a disease wherein various factors deteriorate the stability of the tear layer, resulting in eye discomfort or visual dysfunction, and the disease may be accompanied by eye surface disorder" (NPL 4). Although it is not recommended to use anti-inflammatory agents for dry eye, inflammation is supposed to cause dry eye to develop in the United States. It has been reported that in dry eye, inflammatory cytokines increase in tears and the corneal epithelium, and especially an increase in IFN-y correlates with a decrease in conjunctival goblet cells and dry eye symptoms (NPL 5). The situation as mentioned above has suggested the involvement of inflammation in dry eye under the present conditions.

In the United States and Europe, such as 0.05% cyclosporine (Restasis(R)) eye drops and Lifitegrast (Xiidra(R)), which is an LFA-1 antagonist are used as instillation therapy with anti-inflammatory agent, and the effectiveness thereof has been reported. Unfortunately, these instillation treatments are disadvantageous in that the treatments take time to exhibit an effect, and it is difficult to continue the treatments due to side effects.

### [Citation List]

### [Non Patent Literature]

[NPL 1]
   Clinical Guidelines for Dry Eye (edited by the Clinical Guidelines Preparation Committee of the JAPAN DRY EYE SOCIETY), published on May 10, 2019
[NPL 2]
   Document of the 21st Japanese Ophthalmological Panel Discussion for Journalists, June 2, 2022
[NPL 3]
   N. Yokoi, et. al., American Journal Ophthalmology, 2015, 159, 748-754.
[NPL 4]
   Japanese Revised Definition of Dry Eye and Diagnostic Criteria for Dry Eye (Version 2016), the JAPAN DRY EYE SOCIETY, Committee for Definition of Dry Eye and Diagnostic Criteria for Dry Eye
[NPL 5]
   Stephen C Pflugfelder, et. al., Investigative Ophthalmology & Visual Science, 2015, 56, 7545-7550.

### [Summary of Invention]

### [Technical Problem]

As mentioned above, any fundamental therapeutic agent and any fundamental therapy for dry eye have not been found yet. Improvement in the QOLs (qualities of lives) of patients requires the development of a highly effective therapeutic agent or a highly effective therapy.

The infiltration of lymphocytes and a decrease in goblet cells in lesions are observed in dry eye from a pathological viewpoint, and dry eye exhibits chronic inflammatory pathological condition. The pathogenesis thereof is however still unknown.

It has been revealed that dry eye is often coincident with systemic autoimmune diseases such as Sjogren's syndrome, rheumatism, and systemic lupus erythematosus (Yumiko Shindo, Shigeaki Ono, Japanese Journal of Allergology, 2003, 52, 518-521). Research has been reported a significant rise in inflammation-related molecules such as IFN-y, IL-1β, IL-6, IL-8, and TNF-α in tears of dry eye patients (Matilde Roda, et. al., International Journal of Molecular Sciences, 2020, 21, 3111). Furthermore, it has also been reported that the amount of IFN-γ in tear correlates with symptoms of dry eye (NPL 4). It has also been reported that the amount of IFN-γ in tears can be a biomarker for dry eye (David Charles Jackson, et. al., Investigative Ophthalmology & Visual Science, 2016, 57, 4824-4830). It is conceivable that IFN-y plays a key role in the development of dry eye like other autoimmune diseases. If dry eye model mice are created from IFN-γ knockout mice, the symptoms of dry eye are mild. It has been reported that the administration of IFN-γ to the mice results in a decrease in the number of goblet cells and an increase in CD4-positive T-cell infiltration, which are symptoms of dry eye (Xiabo Zhang, et. al., Investigative Ophthalmology & Visual Science, 2011, 52, 6279-6285). It has also been reported that an anti-IFN-γ-antibody is administered to dry eye model mice to suppress those dry eye symptoms (Xiaobo Zhang, et. al., Experimental Eye Research, 2014, 118, 117-124). These reports suggest that IFN-y plays an important role in pathological condition of dry eye. Any therapeutic agent for dry eye targeting IFN-y has not, however, been known until now.

Previously, as agents that inhibit the action of IFN-y, antibodies and Janus kinase inhibitor have been developed. However, anti-IFN-y antibodies, for example, have some problems, including that (1) since they are biologics, there is a risk of biological contamination and the like, (2) antigenicity becomes a problem in long-term administration, and (3) since they are protein preparations, a cold chain is required for storage and transportation, and they are unsuitable for eye drops, which need to be administered several times daily.

With regard to the problem (2) above, the antibody production rate for common antibody drugs is said to be about 30%. Therefore, when a long-term treatment is required, a case is often observed in which an antibody against another antibody is generated, causing an anaphylactic reaction and making it difficult to continue the treatment.

Furthermore, with regard to the problem (1) above, since serum and other substances are often used in the producing process of biologics, there is concern about the biological risk of contamination by viruses and other contaminants. With regard to the problem (3), the protein preparation always requires handling at a low temperature, which increases the cost of transportation and storage, and also reduces convenience for patients using the preparation.

Four Janus kinase inhibitors including Tofacitinib (product name: Xeljanz^{®}), Baricitinib (product name: Olmient^{®}), Peficitinib (product name: Smyraf^{®}) and Upadacitinib (product name: RINVOQ^{®}) have been applied to and marketed for rheumatoid arthritis, an autoimmune disease. Since these Janus kinase inhibitors are low-molecular-weight compounds that can be produced by chemical synthesis, they are postulated not to have the problems caused by being antibodies described above. On the other hand, multiple subtypes of Janus kinase exist and are activated by binding to the intracellular domains of multiple cytokine receptors including interleukin 2 (IL-2) receptor, interleukin 4 (IL-4) receptor, interleukin 7 (IL-7) receptor, and interferon-α (IFN-α) receptor, as well as the IFN-γ receptor, and transmit receptor signals.

Therefore, Janus kinase inhibitors may inhibit signaling of not only IFN-y but also IL-2, IL-4, IL-7, IFN-α, and the like (Yvan Jamilloux, et. al., Autoimmunity Reviews, 2019, 18, 11, 102390). This is cause for concern about safety in long-term administration, and suggests that unexpected adverse effects may be exhibited due to susceptibility to infection. Since Janus kinase inhibitors require a buffer due to low water solubility thereof, the irritativeness of the buffer itself is also concerned.

The present invention has been completed in view of such a situation, and is directed to providing a dry eye therapeutic agent that can selectively inhibit IFN-γ as a target substance, has no risk of biological contamination, can be stored at room temperature, and is highly soluble and less irritative.

### [Solution to Problem]

In order to solve the above-mentioned problems, a dry eye therapeutic agent containing as an active ingredient a DNA oligonucleotide of the present invention adopts the following aspects.

The first aspect of the present invention provides a dry eye therapeutic agent containing a DNA oligonucleotide having a nucleotide sequence set forth in any of SEQ ID NO:1 to 3 and selectively binding to interferon-y (IFN-y). The DNA oligonucleotide of this aspect exerts a therapeutic effect on dry eye by selectively binding to IFN-y and inhibiting the activity thereof.

The nucleotide sequence set forth in SEQ ID NO:2 is a sequence in which an oligonucleotide comprising natural bases of 9 residues is added to a 3' end of the nucleotide sequence set forth in SEQ ID NO:1.

The nucleotide sequence set forth in SEQ ID NO:3 is a sequence in which a 53rd base from the 5' end of the nucleotide sequence set forth in SEQ ID NO:2 was replaced with any base.

In the first aspect described above of the present invention, a base X in a sequence of the DNA oligonucleotide described above may be an artificially produced base, and the artificially produced base may be chemically modified with a low-molecular-weight compound.

The low-molecular-weight compound in the first aspect described above has a molecular weight of about 200 to 1000. Examples of a candidate therefor include an anti-inflammatory compound selected from glucocorticoid, tacrolimus, sirolimus, cyclosporine, methotrexate and leflunomide.

In the first aspect described above of the present invention, a base X in a sequence of the DNA oligonucleotide having the nucleotide sequence set forth in SEQ ID NO:3 may be an artificially produced base, and the artificially produced base may be chemically modified with a medium-molecular-weight compound, a high-molecular-weight compound, a biopolymer, or a biocompatible polymer. The medium-molecular-weight compound in this aspect has a molecular weight of about 1000 to 20000, and the high-molecular-weight compound in this aspect has a molecular weight of about 20000 to 400000.

The high-molecular-weight compound in the aspect described above may be any biocompatible large molecule with a molecular weight of 20000 or more. Examples of medium-molecular-weight compounds or high-molecular-weight compounds in the present aspect include, but are not limited to, PEGs, bipolar polymers, oligosaccharides, fat-soluble polymers, peptides, oligonucleotides, and antibodies. Antibodies belong to high-molecular-weight compounds, while PEGs, bipolar polymers, oligosaccharides, fat-soluble polymers, peptides, and oligonucleotides belong to medium-molecular-weight compounds or high-molecular-weight compounds, depending on their molecular weight. The molecular weight of a medium-molecular-weight compound or a high-molecular-weight compound is expressed as an average molecular weight defined by the number average molecular weight (Mn) or the weight average molecular weight (Mw).

### [Advantageous Effects of Invention]

The DNA oligonucleotide having the nucleotide sequence according to the present invention selectively binds to INF-y. The DNA oligonucleotide can selectively inhibit the activity of INF-y thereby. Also, according to a dry eye therapeutic agent containing a DNA oligonucleotide having a nucleotide sequence of the present invention, its production is made without a risk of biological contamination with virus and the like, since there is no need to use serum or other substances in its production. In addition, the DNA oligonucleotide having a nucleotide sequence of the present invention can be stored at room temperature, which is advantageous compared to conventional methods in terms of transportation and storage costs and can improve convenience for patients who use it. The dry eye therapeutic agent containing the DNA oligonucleotide having the nucleotide sequence of the present invention can be administered by eye drops due to the molecular weight thereof.

While the DNA oligonucleotide of the present invention does not inhibit the signaling of IL-2, IL-4, IL-7, IFN-α, and the like, the DNA oligonucleotide inhibits only the action of IFN-y. According to the dry eye therapeutic agent containing as an active ingredient the DNA oligonucleotide having a nucleotide sequence of the present invention, unanticipated adverse effects due to susceptibility to infection can thus be reduced compared to Janus kinase inhibitors and other therapeutic agents even when administered for a long period. Further, when compared to anti-IFN-y antibodies, the DNA oligonucleotide of the present invention is less antigenic than antibodies, which makes it an agent that can be used for a long period of time. Furthermore, the DNA oligonucleotide of the present invention has no risk of biological contamination, and can be stored at room temperature.

### [Brief Description of Drawings]

[Fig. 1] Fig. 1 shows a change in the number of hairs on the grafted skin tissue piece before and after the administration of a DNA oligonucleotide of an embodiment of the present invention, with the vertical axis showing the change in the number of hairs per grafted skin tissue piece.
[Fig. 2A] Fig. 2A shows the inhibition of MHC class I expression in the dermal sheath cup by administration of the DNA oligonucleotide of an embodiment of the present invention.
[Fig. 2B] Fig. 2B shows the inhibition of MHC class I expression in the outer root sheath by administration of the DNA oligonucleotide of an embodiment of the present invention.
[Fig. 3A] Fig. 3A shows the inhibition of MHC class II expression in the connective tissue sheath by administration of the DNA oligonucleotide of an embodiment of the present invention.
[Fig. 3B] Fig. 3B shows the inhibition of MHC class II expression in the outer root sheath by administration of the DNA oligonucleotide of an embodiment of the present invention.
[Fig. 4A] Fig. 4A shows the results of the effect of the addition of surrogate aptamer in an embodiment of the present invention to L929 mouse fibroblasts on STAT1 phosphorylation by mouse IFN-y.
[Fig. 4B] Fig. 4B shows the results of the effect of the addition of negative control DNA to L929 mouse fibroblasts on STAT1 phosphorylation by mouse IFN-y.
[Fig. 5] Fig. 5 shows the results of suppression of corneal disorders in an experiment on the preventive effect of a surrogate aptamer of an embodiment of the present invention on a mouse dry eye model.
[Fig. 6] Fig. 6 shows the results of suppression of corneal disorders in an experiment on the therapeutic effect of a surrogate aptamer of an embodiment of the present invention on a mouse dry eye model.
[Fig. 7A] Fig. 7A shows the results of suppression of a decrease in goblet cells in the conjunctiva on the eye surface in an experiment on the therapeutic effect of a surrogate aptamer of an embodiment of the present invention on a mouse dry eye model.
[Fig. 7B] Fig. 7B shows the results of suppression of CD4-positive T-cell infiltration into the conjunctiva on the eye surface in an experiment on the therapeutic effect of a surrogate aptamer of an embodiment of the present invention on a mouse dry eye model.

### [Description of Embodiments]

Hereinafter, embodiments of a dry eye therapeutic agent will be described containing as an active ingredient an DNA oligonucleotide of the present invention.

With regard to a drug that targets IFN-y, Emapalumab, an anti- IFN-y antibody, has been approved by FDA in 2018 for hemophagocytic lymphohistiocytosis, which is an intractable autoimmune disease until now, and it is marketed under the trade name Gamifant.

However, as described above, anti-IFN-y antibodies have problems such as biological contamination and other risks due to being biologics, antigenicity in long-term administration, unsuitability for topical administration including transdermal administration or transmucosal administration due to the high molecular weight thereof, and conditions for storage and transportation due to being protein preparations. Therefore, the creation of a therapeutic agent has been desired that solves these problems and inhibits IFN-y effectively.

Therefore, the present inventors attempted to develop an IFN-y inhibitor using the DNA oligonucleotide of the present invention as a DNA aptamer as a means to solve the problems described above. The DNA aptamer is a ligand molecule which forms a secondary structure or a tertiary structure of a single-stranded DNA oligonucleotide by forming a complementary strand between complementary sequences in the DNA oligonucleotide molecule, and binds specifically and strongly to a target molecule by its steric structure. While the binding of the DNA aptamer having a specific sequence can inhibit or suppress the activity of target molecules, the binding also enables the enhancement thereof. Although DNA aptamers are smaller than antibodies, with a molecular weight of about one-tenth or less than that of antibodies, they have high compatibility and high target selectivity comparable to those of antibodies. Therefore, an IFN-y inhibitor using a DNA aptamer can minimize the occurrence of adverse effects due to off-target. It is postulated that DNA aptamers are a suitable modality as a means to solve the problem, since DNA aptamers can be produced through chemical synthesis. As used herein, "selectively binds to IFN-y" includes strong and specific binding of the DNA oligonucleotide of the present embodiment as a DNA aptamer to IFN-y, which is a target substance.

Since DNA aptamers form more compact size three-dimensional structures than antibodies, DNA aptamers enable topical administration such as eye drops, transdermal administration or transmucosal administration.

DNA aptamers have the following advantages and are expected to be useful: (1) with a relatively small molecular weight, administration by eye drops, transdermal formulations such as ointments and patches or transmucosal formulations may be possible; (2) as chemically synthesized products, a risk of biological contamination is low; (3) generally antigenicity is low; (4) due to being DNA, sufficient stability is secured at room temperature under nuclease-free and almost neutral conditions; and (5) with almost no inhibitory activity to cytochrome P450, a drug-metabolizing enzyme, there is no effect on concomitant agents, and the like. In addition, DNA aptamers do not have a problem that an antibody is generated against antibody drugs, the problem that occurs when long-term treatment with the antibody is necessary, and therefore, long-term administration is possible.

As a specific means to treat diseases using the DNA aptamer, the means is assumed to treat autoimmune diseases or diseases considered to result mainly from the overproduction of IFN-γ by neutralizing IFN-y through administration of the aptamer itself or a modified form of the aptamer.

The present inventors have found a DNA aptamer having a sequence of SEQ ID NO: 1 in Table 1 including two artificial bases, in the nucleotide sequence, that are Ds (7-(2-thienyl)imidazo[4,5-b]pyridine) as a DNA aptamer that can bind to human IFN-y with high affinity to inhibit the activity thereof.

Even though the DNA aptamers exhibit strong activity *in vitro,* the DNA aptamers does not necessarily express activity *in vivo* for the following reason: DNA aptamers are rapidly decomposed with nuclease in biological tissue. Accordingly, the present inventors have found that a DNA aptamer having a nucleotide sequence of SEQ ID NO: 2 in Table 1 wherein a natural nucleotide sequence of nine residues is bound to the 3' end of the nucleotide sequence of SEQ ID NO: 1 has nuclease-resistance and is stable in biological tissue. It was confirmed that the DNA aptamer having the nucleotide sequence of SEQ ID NO: 2, including two Ds, exhibits efficacy in an autoimmune humanized mouse alopecia areata model, into which human scalp tissue is transplanted, is stable in biological tissue, and can inhibit IFN-y (Japanese Patent Application 2021-166794). In the sequence set forth in SEQ ID NO: 2, the 53rd base from the 5' end is substituted with any base, X, to obtain a sequence of SEQ ID NO: 3. Polyethylene glycol (PEG) is added to the base X to obtain a PEG-modified form of a DNA aptamer having the sequence of SEQ ID NO: 3. It was confirmed by SPR (surface plasmon resonance) that the PEG-modified form is capable of binding to IFN-γ. This has shown that the modification of the base, X, does not affect the IFN-γ-binding activity of the DNA aptamer having a sequence of SEQ ID NO: 3, namely that the DNA aptamer having the sequence of SEQ ID NO: 3 has IFN-y inhibitory activity equivalent to that of the DNA aptamer having the sequence of SEQ ID NO: 2.

In the present embodiment, the DNA oligonucleotides having the nucleotide sequences listed in Table 1 are used as the DNA aptamers. The present embodiment comprises the use of the DNA oligonucleotides having the nucleotide sequences listed in Table 1 as the dry eye therapeutic agents.

**[Table 1]**

| SEQ ID NO: | Sequence (5' → 3') |
|---|---|
| 1 | CCCGCCCGGGTCCGCGAAGCGGTAGGT**Ds**TGGGCTAGGC**Ds**GCTGGCGGG |
| 2 | CCCGCCCGGTCCGCGAAGCGGTAGGT**Ds**TGGGCTAGG**Ds**GCTGOUGUGGCGCGAAGCA |
| 3 | CCCGCCCGGGTCCGCGAAGCGGTAGG**Ds**TGGGCTAGGC**Ds**GCTGGCGGGCGCGXAGCG |
| 4 | GGCCGGTACCCGA**Ds**CCACAGTTTAT**Ds**GTTGTACTAGTTTTGCAGGGTCTGGCCCGCGAAGCG |

The nucleotide sequence set forth in SEQ ID NO:2 of Table 1 is a sequence in which an oligonucleotide comprising natural bases of 9 residues (5'-CGCGAAGCG-3') (mini-hairpin sequence) is added to the 3' end of the sequence set forth in SEQ ID NO:1. The nucleotide sequence set forth in SEQ ID NO:3 of Table 1 is a sequence in which the 53rd base from the 5' end of the sequence set forth in SEQ ID NO:2 was replaced with any base X. The X represents any natural base, any non-natural base or a modified base, or a modified base to which a low-molecular-weight compound, a peptide, an oligonucleic acid, an oligosaccharide, a protein or the like, or a high-molecular-weight compound used in vivo (biopolymer) or a biocompatible polymer is bound. Details of the nucleotide sequence of SEQ ID NO: 4 will be described below.

Examples of the high-molecular-weight compound to be bound to the modified base include polyethylene glycol (PEG) having a molecular weight of 20000 or more and any biocompatible large molecule having a molecular weight of 20000 or more. A biocompatible polymer is a chemically synthesized compound that is not normally used in vivo and safe enough to be placed in vivo without causing inflammation or toxic reactions. Examples of medium-molecular-weight compounds include peptides, oligonucleic acids, oligosaccharides, proteins, PEGs, and any biocompatible polymers with a molecular weight of 1000 or more and less than 20000.

As functional groups for modification, azido group (-N₃), amino group (-NH₂), carboxyl group (-COOH) or its active ester, alkynyl group (-CC) or a cyclic structure having an alkynyl structure, formyl group (-CHO), hydrazide group (-NH-NH₂), hydroxyl group (-OH), thiol group (-SH), cyano group (-CN), vinyl group (-CHCH₂) and maleimide group can be used.

As used herein, "natural base" refers to either adenine, guanosine, cytosine, or thymine. As used herein, "non-natural base" refers to a base that is artificially synthesized having properties similar to the natural base, and is sometimes referred to as "artificial base" herein. As used herein, "modified base" refers to a base to which a side chain structure with one or more functional groups activated for modification is added, and is a kind of "artificially produced base". Examples of modifications include methylation, deamination, atomic place exchange, thiolation of oxygen in phosphate site, and introduction of a water-soluble or fat-soluble substituent group into the base portion of a natural base. Specifically, the examples include modified pyrimidines, modified purines, and other heterocyclic bases. Ds in SEQ ID NO:1 to 3 represents 7-(2-thienyl)imidazo[4,5-b]pyridine, an artificial base. As an artificial base, in addition to Ds itself, a base with a side chain introduced into Ds may be used. Hereafter, in the present embodiment, a DNA aptamer having a sequence set forth in SEQ ID NO:1, 2, 3, or 4 of Table 1 will be referred to as "Aptamer 1", "Aptamer 2", "Aptamer 3" or "Aptamer 4" respectively.

With respect to the usefulness of the DNA aptamer of the present embodiment, it was observed herein that when a DNA aptamer listed in Table 1 (aptamer 2) was injected intradermally into the transplanted skin of an immune-tolerance mouse of autoimmune hair loss model which was transplanted with human scalp tissue piece, regeneration of hair was promoted, resulting in suppression of further hair loss (Example 4). Details will be described later.

Pathological analysis of the mechanism of activity expression of Aptamer 2 in this model revealed that Aptamer 2 almost completely inhibited expression of MHC class I and II. Detailed results will be described later. That is, it is postulated that by inhibiting activity of IFN-y, Aptamer 2 inhibited production of MHC class I and II, the source of expression of autoimmunity, to improve autoimmunity, thus improving the symptoms of alopecia areata. This indicates that aptamer 2 is effective not only against alopecia areata but also against of dry eye, presumed to result partly from overproduction of IFN-y that is the target of the present invention, and that providing an IFN-y inhibitor containing the DNA aptamer could be a means to solve the problems.

It needs to be confirmed in a disease animal model before the certification of the efficacy of DNA aptamers in humans whether the DNA aptamers that inhibit the activity of IFN-γ are effective in the treatment of dry eye. It is known that a mouse dry eye model (model in which dry eye is induced by a dry environment at a humidity of 20% and the administration of scopolamine) is used for various purposes as an animal model that has been establish until now, and is similar in pathological condition of dry eye to humans (Terry G. Coursey, et. al., Translational Vision Science & Technology, 2018, 7, 24: hereinafter referred to as "Reference Literature 1"). Unfortunately, the above-mentioned DNA aptamers that specifically bound to human IFN-y did not exhibit binding activity to mouse IFN-y. The efficacy of the above-mentioned DNA aptamers cannot, therefore, be confirmed in the mouse model.

Accordingly, the present inventors have examined the acquisition of an aptamer that is highly similar in physical properties to the above-mentioned DNA aptamers, and binds to mouse IFN-y to inhibit the activity thereof as a surrogate aptamer.

The surrogate aptamer needs to satisfy the following criteria to be highly similar in physical properties to the above-mentioned DNA aptamers.
(1) The aptamer is a DNA aptamer.
(2) The aptamer contains two artificial bases, Ds, in the nucleotide sequence thereof, and has natural bases as the other bases.
(3) The number of bases of the aptamer is within the range of 57 residues ± 10% (51 to 62 residues).
(4) The aptamer has a mini-hairpin sequence of nine residues on the 3' end of the nucleotide sequence thereof.

Since the aptamer satisfying the above-mentioned criteria is similar in structure to the above-mentioned DNA aptamers, the aptamer is assumed to be also similar in physical properties.

A search for the surrogate aptamer satisfying the above-mentioned criteria enables obtaining a DNA aptamer having a sequence listed in SEQ ID NO: 4 of Table 1, having residues with 62 bases, containing two Ds, and having a mini-hairpin sequence on the 3' end thereof (aptamer 4) (Example 5). Details thereof will be described below. The obtained DNA aptamer had a KD value of 2.47 nM, which indicates the capability to bind to mouse IFN-y, and is around one hundredth as high as the capability of the aptamer 2 to bind to human IFN-y (KD value: 33 pM). The aptamer having five times the molarity of mouse IFN-y was added to the mouse IFN-y to inhibit mouse IFN-γ competitively, resulting in suppressing the activity of mouse IFN-y almost completely. This result confirmed that the DNA aptamer obtained by the search has sufficient activity to function as a surrogate aptamer.

Dry eye was induced by a dry environment at a humidity of 20% and the administration of scopolamine to create dry eye mice (Reference Literature 1). The obtained surrogate aptamer was ophthalmically administered to the dry eye mice at the same time as the induction of dry eye or from three days after the induction of dry eye. Consequently, the surrogate aptamer suppressed a rise in the corneal disorder score of the mice compared to the non-administration group (Examples 6 and 7).

The results of evaluating the ocular tissue histopathologically showed that the aptamer-treated group was high in the density of goblet cells in the conjunctiva on the eye surface compared to the non-administration group, and was not different therein from the normal group. Furthermore, it was shown that the aptamer-treated group was low in the density of CD4-positive T-cells that infiltrated into the conjunctiva on the eye surface compared to the non-administration group, and was not different therein from the normal group. The above showed that the administration of the surrogate aptamer suppressed inflammation in the conjunctiva on the eye surface (Example 7).

Since the DNA aptamer of the present embodiment exhibits the inhibitory action on human IFN-y in human tissue, and is highly similar in physical properties to the surrogate aptamer, it has been inferred that the DNA aptamer has the action of suppressing the development of dry eye in humans, and is useful as the dry eye therapeutic agent like the surrogate aptamer.

As a DNA aptamer, DNA oligonucleotides having any of the sequences listed in Table 1 can be used as they are, or it is also possible to use those modified at a site that does not affect activity of the DNA aptamer. Examples of the modified form of the DNA aptamer include a DNA aptamer chemically bound to a medium-molecular-weight or high-molecular-weight compound such as PEG, a peptide, or an oligonucleotide, a polymer into which the same DNA aptamers are formed by a chemical process, and a DNA aptamer having a partially converted or modified sequence. When a DNA oligonucleotide of the present embodiment is modified and used as a DNA aptamer, the base portion is preferred as the modification portion. An artificial base or a modified base can be modified using existing methods, and the 3' and 5' ends can also be modified.

PEG-modified forms of DNA aptamers are utilized for improving Pharmacokinetics (PK)-Pharmacodynamics (PD) Profile that is commonly used for proteins, peptides, oligonucleic acids including aptamers to achieve improved pharmacokinetics, and a number of PEG-modified aptamers have been developed. It is known that if binding activity of a PEG-modified aptamer to a target protein is retained, it shows the same level of activity in the body as the aptamer before PEG modification, and there is almost no toxic expression due to the PEG modification (C. Simone Fishburn, Journal of Pharmaceutical Sciences, 2008, 97, 4167-4183; Katarina D. Kovacevic, et. al., Advanced Drug Delivery Reviews, 2018, 134, 36-50).

As drug formulations for systemic administration, formulations can be prepared as an injectable formulation in vials containing lyophilized powders, vials containing aptamer solution, and pre-filled syringes.

The DNA aptamer of the present embodiment can be produced as a preparation for inhalation by placing a nanoparticle adsorbing or containing the DNA aptamer or a solution thereof, or a powder of the DNA aptamer granulated to an appropriate size with a granulating material in an inhalation device.

The DNA aptamer of the present embodiment can be used as an eye drop by dissolving it as it is in a suitable solvent such as a buffer solution utilizing its high water-solubility.

Examples of conceivable methods for topical administration include a method for transmucosal administration. The DNA aptamers of the present embodiment are dissolved in a solvent such as a highly biocompatible buffer solution to be available as an agent to be transmucosally administered such as an agent to be intravesically administered.

As preparations for injection, preparations for inhalation and eye drops, it is possible to use them in a form that the DNA aptamer of the present embodiment is encapsulated or bonded in nanoparticles such as fatty nanoparticles, nanoparticles of biodegradable polymers such as PLGA (Polylactic-co-Glycolic Acid), gold nanoparticles, and then dispersed or dissolved in physiological saline solution, physiological buffer solution, and the like.

The DNA aptamer of the present embodiment can be applied as a topical transdermal agent such as a solution, an ointment, a cream, a lotion, a milky lotion, an emulsion, a gel, a biodegradable microneedle, or a poultice.

In the process of producing a transdermal administration formulation, as an absorption enhancer, lower alcohols such as ethanol, polyhydric alcohols such as ethylene glycol, fatty acids, esters such as ethyl acetate, surfactants, and ionic liquids and the like may be used. For the production of the transdermal administration formulation, production process in which biodegradable polymers such as polylactic acid or liposomes are used for making nanoparticles is applicable, and these processes can be combined as appropriate depending on purposes.

The DNA aptamer of the present embodiment can also be used as an administration preparation using a device compatible with physical transdermal absorption enhancement methods such as lontophoresis, Electroporation, Thermalporation, Sonophoresis, a Microneedle array patch, a Needleless syringe, and a micropump.

Since the DNA aptamer of the present embodiment can inhibit IFN-γ selectively, the DNA aptamer is available as a research reagent for experiment in which IFN-y is involved. For example, a test in which the DNA aptamer of the present embodiment is made to act enables evaluating and examining the probability that IFN-y is involved in a noticed physiological phenomenon regardless of whether the physiological phenomenon occurs *in vitro* or *in vivo* to consider the cause of the physiological phenomenon. The DNA aptamer of the present embodiment is added as a reagent to cell culture medium or administered to animals to be available for a wide variety of tests including reaction systems in which IFN-y is inhibited.

### Example 1: Synthesis of DNA aptamer

Aptamer 1 and aptamer 2 were chemically synthesized by the methods described in PCT International Publication No. WO 2013/073602 and PCT International Publication No. WO 2016/143700.

### Example 2: Example of synthesis of aptamer 3

Aptamer 3 was synthesized by introducing Amino-Modifier C6-dT Amidite to the position of X in the sequence of SEQ ID NO:3 using the methods described in PCT International Publication No. WO 2013/073602 and PCT International Publication No. WO 2016/143700. For other substituents of X, they can be synthesized by using commercially available artificial base or modified base amidites.

### Example 3: Synthesis of PEG-modified DNA aptamer

With the base of X produced in Example 2, Aptamer 3 (1 eq) having a primary amine side chain and commercially available NHS-PEG (40000) (1.5 eq) were mixed in a phosphate buffer at pH 7 to 8 and the mixture was stirred for 1 day at room temperature. The reaction solution was concentrated and the resulting modified form was purified by reversed phase HPLC to obtain a PEG-modified form of Aptamer 3. It was confirmed that the obtained PEG-modified form of Aptamer 3 retained its ability to bind to IFN-y using a SPR (Surface Plasmon Resonance).

### Example 4: Confirmation of therapeutic effect using humanized mouse model with alopecia areata

### Step 1 Creation of humanized mouse model with alopecia areata

A humanized model mouse with alopecia areata induced by an intradermal injection of human activated lymphocytes into the scalp tissue transplanted into the mice was created based on the method described in A. Gilhar, et. al., Journal of Investigative Dermatology, 2013, 133, 844-847.

### Step 2

The humanized model mouse with alopecia areata created were divided into three groups, and each group was treated with Vehicle (PBS), Dexamethasone + Minoxidil (Positive control), and Aptamer 2. To the Vehicle group, 15 µL of PBS was intradermally administered to the transplanted skin once every 2 days. To the group treated with aptamer 2 (hereafter referred to as the "aptamer-treated group"), a 15 µL of aptamer 2 solution in PBS was intradermally administered to the transplanted skin once every 2 days, and the concentration of the aptamer 2 solution was gradually increased from 12 nM to 300 nM over 143 days. To the group treated with Dexamethasone + Minoxidil, 40 µL of administration solution containing 2 mg of Dexamethasone and 5% Minoxidil was applied to the transplanted skin once daily.

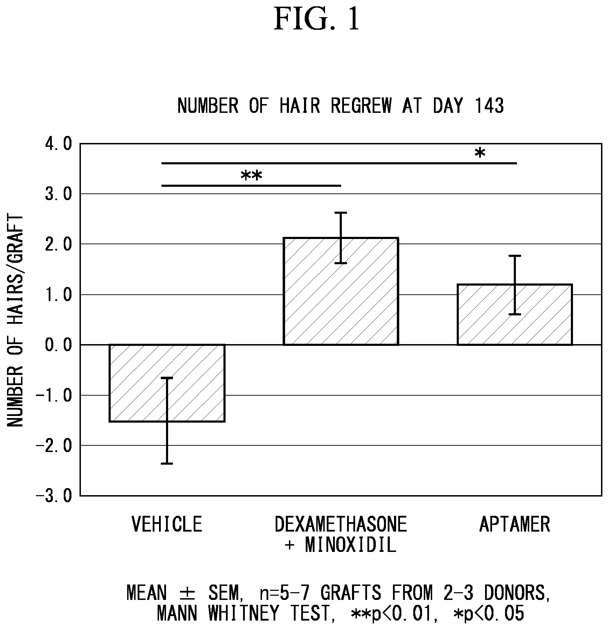

The results after administration in Example 4 are shown in Fig. 1. Fig. 1 shows a change in the number of hairs on the grafted skin tissue piece before and 143 days after the administration, with the vertical axis showing the change in the number of hairs per grafted skin tissue piece. In the Vehicle group ("Vehicle" in Fig. 1), further hair loss progressed during the PBS administration period, while in the Positive control group ("Dexamethasone + Minoxidil" in Fig. 1) and the Aptamer-treated group ("Aptamer" in Fig. 1), further hair loss was suppressed and also hair regeneration was observed.

Pathological analysis results of the hair follicle tissue showed a significant inhibition of CD8-positive T-cell infiltration in the Positive control group and the Aptamer-treated group. This suggests that in the Positive control group and the Aptamer-treated group, inhibition of inflammatory reaction suppressed hair loss progression and promoted hair regeneration.

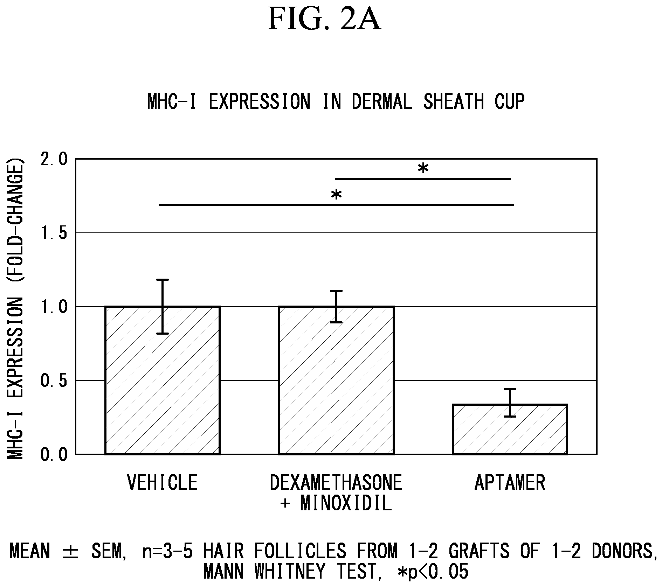

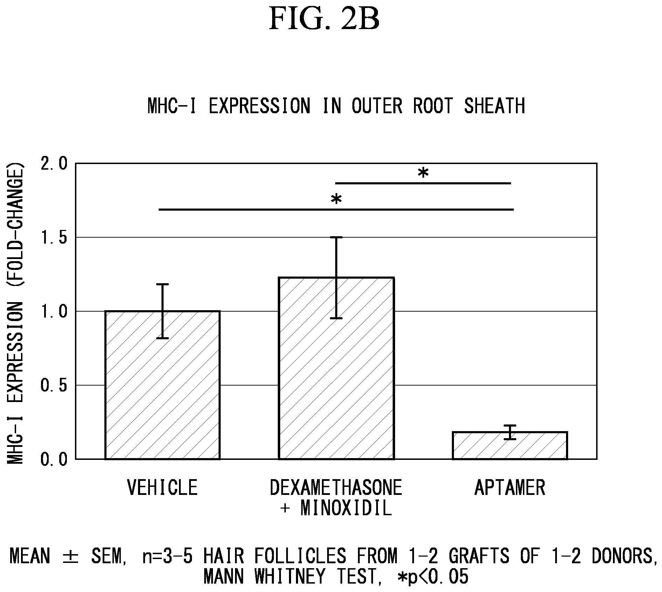

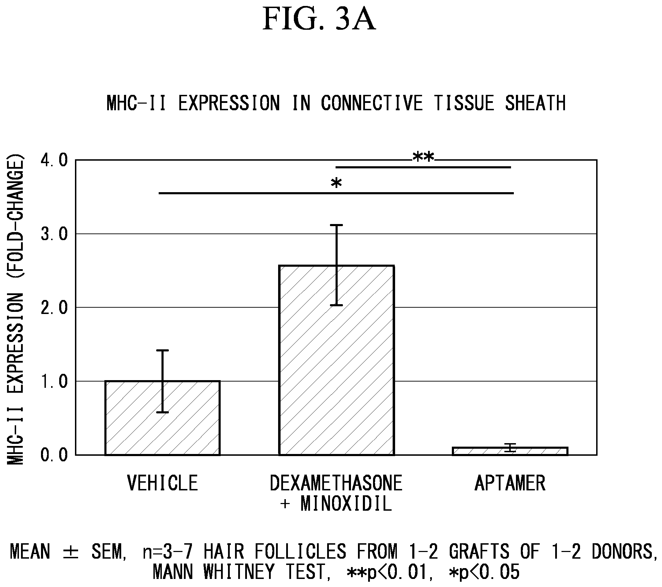

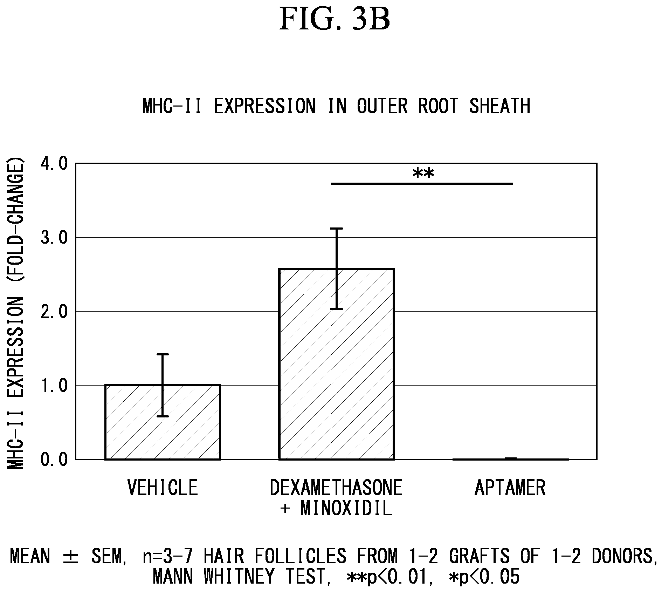

In addition, the expression of MHC was examined by the pathological analysis of the hair follicle tissue after the administration in Example 4. Fig. 2A shows the results of expression of MHC class I in the dermal sheath cups. Fig. 2B shows the results of expression of MHC class I in the outer root sheaths. Fig. 3A shows the results of expression of MHC class II in the connective tissue sheaths. Fig. 3B shows the results of expression of MHC class II in the outer root sheaths. The vertical axis shows the expression levels of MHC class I (Fig. 2A and Fig. 2B) or class II (Fig. 3A and Fig. 3B) as relative values to the expression level in the respective Vehicle group ("Vehicle" in Fig. 2A, Fig. 2B, Fig. 3A and Fig. 3B) as 1.

Inhibition of expression of both MHC class I and II was observed only in the Aptamer-treated group ("Aptamer" in Fig. 2A, Fig. 2B, Fig. 3A and Fig. 3B). This indicates that the mechanism that suppressed inflammation and promoted hair regeneration was different between the Positive control group ("Dexamethasone + Minoxidil" in Fig. 2A, Fig. 2B, Fig. 3A and Fig. 3B) and the Aptamer-treated group. It is assumed that the Positive control group showed direct suppression of inflammation by activation of glucocorticoid receptors, while the Aptamer-treated group suppressed inflammation by inhibiting the production of MHC class I and class II, which cause autoimmunity. In other words, in the Aptamer-treated group, hair follicle tissues recovered from the breakdown of immune tolerance, suggesting that a more fundamental therapeutic effect was obtained. This result suggests that the DNA aptamer of the present embodiment may be able to suppress inflammatory reactions not only in autoimmune skin diseases, but also in autoimmune diseases that occur in other tissues and in diseases such as Hunner-type interstitial cystitis resulting mainly from the overproduction of IFN-y through similar mechanisms.

It was confirmed that the use of the therapeutic agent containing the DNA aptamer of the present embodiment can suppress hair loss and also promote hair regeneration. In addition, as a result of the pathological analysis, it was able to be confirmed that the administration of the DNA aptamer of the present embodiment can almost completely inhibit expression of MHC class I and II.

### Example 5: Production of mouse IFN-y aptamer (surrogate aptamer)

### Step 1

The aptamer was obtained by SELEX targeting mouse IFN-y. The obtained mouse IFN-y aptamer is a DNA aptamer having a sequence listed in SEQ ID NO: 4, having residues with 62 bases, containing two Ds, and having a mini-hairpin sequence on the 3' end thereof (Aptamer 4 in Table 1). The capability to bind to mouse IFN-was measured γ by SPR, so that it was confirmed that the KD value was 2.47 nM.

### Step 2

It was verified that the obtained aptamer inhibited the activity of mouse IFN-y. First, 2 ng/mL mouse IFN-y and each of the aptamer 4 solutions at various molarity were simultaneously added to L929 mouse fibroblasts, followed by incubation at 37°C for 15 minutes. It was then confirmed by flow cytometry using an anti-phosphorylated STAT1 antibody that aptamer 4 inhibited STAT1 phosphorylation.

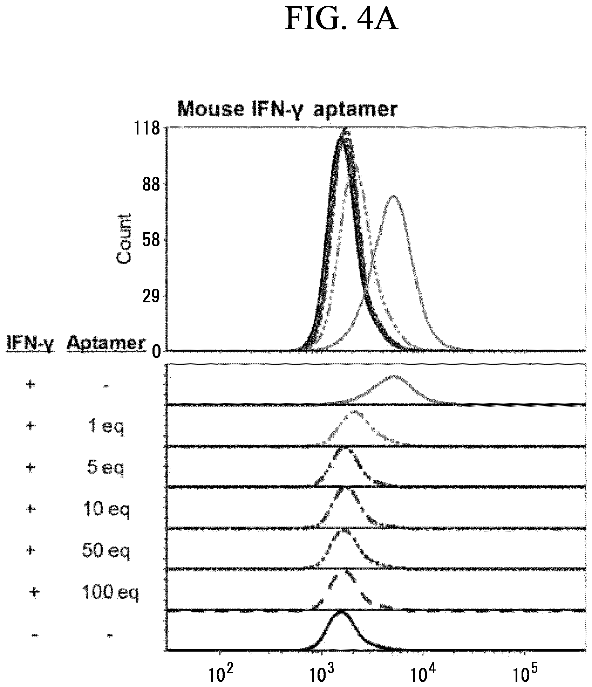

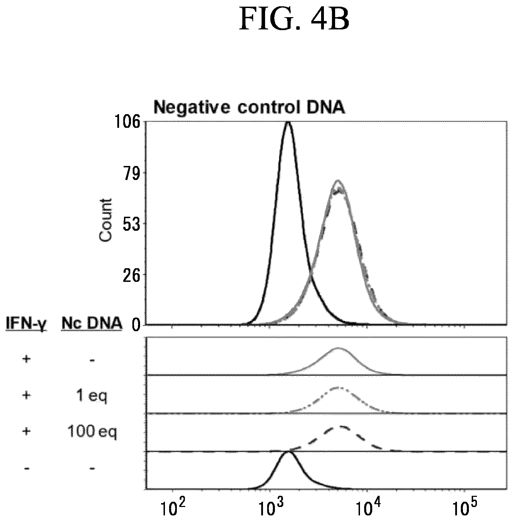

Figs. 4A and 4B show the results. Fig. 4A shows the results of addition of aptamer 4 to L929 mouse fibroblasts. Fig. 4B shows the results of addition of negative control DNA to L929 mouse fibroblasts. In Fig. 4A, the term "Aptamer" means the surrogate aptamer of the present embodiment. In Fig. 4B, the term "Nc DNA" means the negative control DNA. In Figs. 4A and 4B, the terms "1 eq", "5 eq", "10 eq", "50 eq", and "100 eq" indicate the ratios of the molarity of the aptamer to the molarity of IFN-y (for example, 100 eq indicates that aptamer:IFN-y = 100:1). Aptamer 4 was added to mouse IFN-y at five times the molarity of the IFN-y to inhibit the phosphorylation of STAT1 almost completely (Fig. 4A). Meanwhile, even though the negative control DNA was added to IFN-y at 100 times amount of mouse IFN-y in the system to which the negative control DNA was added, the phosphorylation of STAT1 was not inhibited (Fig. 4B). This result confirmed that the obtained aptamer 4 inhibited the activity of mouse IFN-y, and had sufficient activity as a surrogate aptamer.

### Example 6: Examination of preventive effect of the surrogate aptamer on mouse dry eye model

### Step 1 Creation of dry eye mice

Dry eye was induced by a dry environment and the administration of scopolamine to create dry eye mice (Terry G. Coursey, et. al., Translational Vision Science & Technology, 2018, 7, 24). Normal mice are placed in a cage at a humidity of 20% and subjected to dryness stress, and 0.5 mg/mL scopolamine hydrobromide hydrate is intraperitoneally administered to the mice four times daily to dry the eye surface, resulting in corneal epithelial disorder and inflammation of the corneal/conjunctival epithelium.

### Step 2

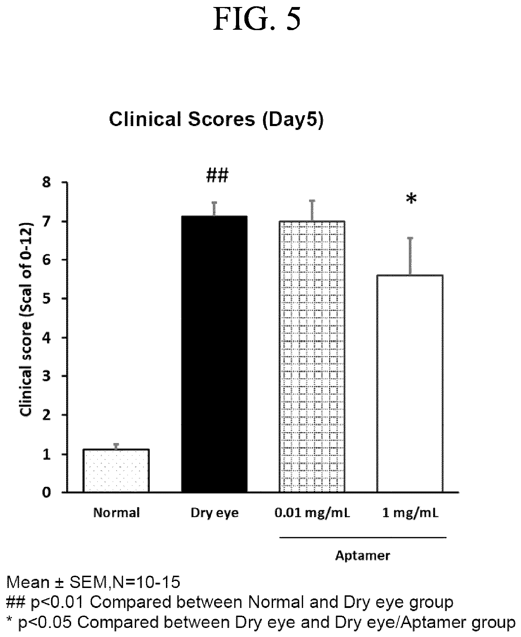

The surrogate aptamer was ophthalmically administered to both eyes of the mice three times daily for five days from the start date of the dry eye induction (0.01 mg/mL or 1 mg/mL, 3 µL/time). A non-administration group (dry eye mice) was used as a control group of dry eye mice. A Normal group (normal mice) was used as a control group of normal mice. The surrogate aptamer was not administered to either of the control groups. Oregon Green dextran, which was a fluorescein derivative, was ophthalmically administered to the mice of the groups five days after dry eye induction, and the eyes were washed one minute after, followed by scoring the Oregon Green dextran remaining on the corneal epithelium to evaluate corneal epithelial disorder. Fig. 5 shows the results of the evaluation of corneal epithelial disorder. While the non-administration group (dry eye mice) significantly increased in the corneal epithelial disorder score compared to the Normal group (normal mice), the aptamer-treated group (1 mg/mL) exhibited a low corneal epithelial disorder score compared to the non-administration group. This showed that corneal epithelial disorder in the mice was further suppressed in the aptamer-treated group than in the non-administration group, and the aptamer exhibited the effect of suppressing the development of dry eye.

### Example 7: Examination of therapeutic effect of surrogate aptamer on mouse dry eye model

### Step 1

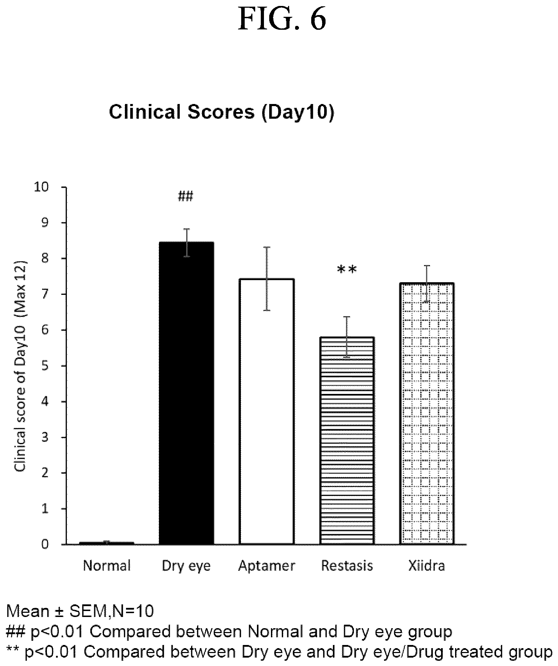

Dry eye was induced in mice by the same method as in Step 1 of Example 6. To the dry eye mice were ophthalmically administered the surrogate aptamer, or Restasis or Xiidra, which were existing drugs, as comparative controls, three times daily from three days after the dry eye induction for seven days (1 mg/mL, 3 µL/time). A non-administration group (dry eye mice) was used as a control group of dry eye mice. A Normal group (normal mice) was used as a control group of normal mice. Oregon Green dextran, which was a fluorescein derivative, was ophthalmically administered to the mice of the groups ten days after the dry eye induction (namely after the end of seven-day administration of the surrogate aptamer or the existing drugs), and the eyes were washed one minute after, followed by scoring the Oregon Green dextran remaining on the corneal epithelium to evaluate corneal epithelial disorder. Fig. 6 shows the results of the evaluation of corneal epithelial disorder. While the non-administration group (dry eye mouse) significantly increased in the corneal epithelial disorder score compared to the Normal group (normal mice), the aptamer-treated group exhibited a low corneal epithelial disorder score in the same way as the Xiidra-treated group, which was one of the existing drug-treated groups, compared to the non-administration group (dry eye mice). This showed that corneal epithelial disorder in the mice was further improved in the aptamer-treated group than in the non-administration group, and the aptamer exhibited a therapeutic effect on dry eye.

### Step 2

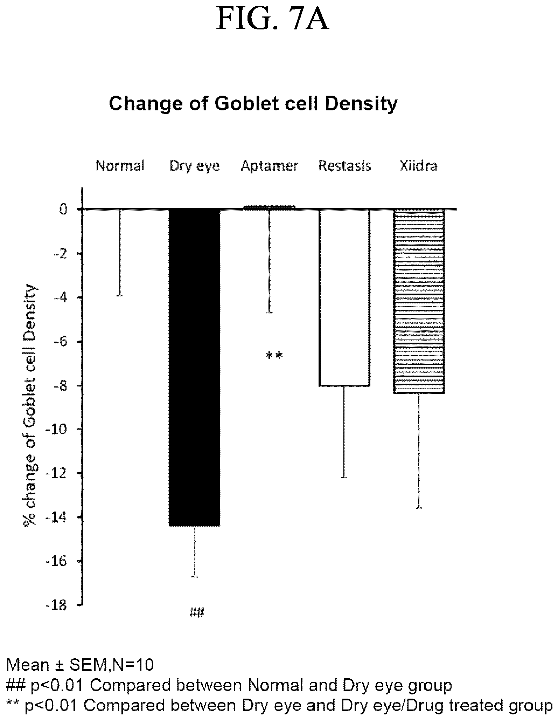

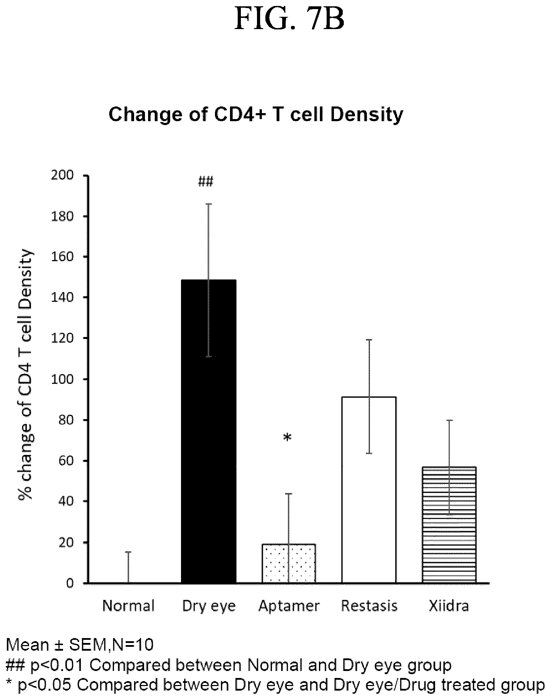

The eyes were extracted after the evaluation of corneal epithelial disorder (ten days after the dry eye induction, namely after the end of the seven-day administration of the surrogate aptamer or the existing drugs). The corneal/conjunctival epithelia were histopathologically evaluated. Slices prepared from the mouse ocular tissues ten days after the dry eye induction were stained with periodic acid-Schiff (PAS) for evaluation. Fig. 7A shows the results of the evaluation of the goblet cell density of the conjunctival epithelia. While the non-administration group (dry eye mice) significantly decrease in goblet cell density stained with PAS compared to the Normal group (normal mice), the aptamer-treated group was equivalent in goblet cell density to the Normal group (normal mice). Although a decrease in goblet cell density was also suppressed in the existing drug (Restasis or Xiidra)-treated groups compared to the non-administration group, the inhibitory effects thereof were weaker than that of the aptamer-treated group. Furthermore, Fig. 7B shows the results of evaluating the ocular tissue slices by immunohistological staining with an anti-CD4 antibody. While the non-administration group (dry eye mice) significantly increased in CD4-positive T-cell infiltration into the conjunctival epithelium compared to the Normal group (normal mice), CD4-positive T-cell infiltration was suppressed in the aptamer-treated group. Although CD4-positive T-cell infiltration was also suppressed in the existing drug (Restasis or Xiidra)-treated groups compared to the non-administration group, the inhibitory effects thereof were weaker than that of the aptamer-treated group.

It is postulated that the results in Examples 4 and 6 above may have resulted from the strong inhibition of IFN-y activity by the DNA aptamer of the present embodiment. Therefore, the DNA aptamer of the present embodiment can be used to provide the most unprecedented and effective therapeutic agent and treatment method for autoimmune diseases, including alopecia areata, and diseases such as dry eye resulting mainly from the overproduction of IFN-y.

From the results of the above Examples, it is also postulated that the DNA aptamer of the present embodiment binds to IFN-y with high specificity. When compared to Janus kinase inhibitors (Yvan Jamilloux, et. al., Autoimmunity Reviews, 2019, 18, 102390), which by the pharmacological nature inevitably inhibit multiple cytokine signals, the DNA aptamer of the present embodiment, which selectively inhibits IFN-y activity, can reduce the likelihood of development of adverse effects.

DNA aptamers are generally less likely to produce anti-DNA aptamer antibodies. This allows the DNA aptamer of the present embodiment to be administered over a long period of time in the treatment of chronic inflammatory diseases.

The DNA aptamer of the present embodiment can be produced by chemical synthesis. Therefore, it can be provided as a safe agent with stable quality and low risk of biological contamination.

The DNA aptamer of the present embodiment can be produced at a lower cost than biologics. In addition, while biologics require low-temperature conditions for their storage and the transportation, DNA aptamers are stable at room temperature. Therefore, a cold chain is not necessarily required in the transportation and storage of the drug formulation containing the DNA aptamer of the present embodiment.

By making the DNA aptamer of the present embodiment into a transdermal administration formulation, a therapeutic agent which is not invasive in administration, is of less concern for adverse effects and is easy to use can be provided.

The therapeutic agent containing the DNA aptamer of the present embodiment can be indicated for systemic autoimmune diseases and diseases such as dry eye resulting mainly from the overproduction of IFN-γ by systemically administering it as an injectable formulation. In addition, when made into an injectable formulation, it can be a drug formulation which is easy to use for patients, such as a pre-filled syringe which can be stored at room temperature.

## Claims

1. A dry eye therapeutic agent comprising as an active ingredient a DNA oligonucleotide having a nucleotide sequence set forth in any of SEQ ID NO:1 to 3 and selectively binding to interferon-y (IFN-y).

2. The dry eye therapeutic agent according to claim 1, wherein a base, X, in a sequence of the DNA oligonucleotide having the nucleotide sequence set forth in SEQ ID NO: 3 is an artificially produced base, and the artificially produced base is chemically modified with a low-molecular-weight compound.

3. The dry eye therapeutic agent according to claim 2, wherein the low-molecular-weight compound is an anti-inflammatory compound selected from glucocorticoid, tacrolimus, sirolimus, cyclosporine, methotrexate and leflunomide.

4. The dry eye therapeutic agent according to claim 1, wherein a base X in a sequence of the DNA oligonucleotide having a nucleotide sequence set forth in SEQ ID NO:3 is an artificially produced base, and the artificially produced base is chemically modified with a medium-molecular-weight compound, a high-molecular-weight compound, a biopolymer, or a biocompatible polymer.

5. The dry eye therapeutic agent according to claim 4, wherein the high-molecular-weight compound is a polyethylene glycol (PEG) with a molecular weight of 20000 or more or any biocompatible large molecule with a molecular weight of 20000 or more.
